# EUROPÄISCHE PATENTSCHRIFT

(11) **EP 1 115 338 B1**
(45) Veröffentlichungstag und Bekanntmachung des Hinweises auf die Patenterteilung: **16.08.2006**
(21) Anmeldenummer: 00912604.6
(22) Anmeldetag: 15.03.2000
(51) Int. Cl.: A61B 17/16

(54) **ROTIERENDES CHIRURGISCHES WERKZEUG**
ROTATING SURGICAL INSTRUMENT
OUTIL CHIRURGICAL A ROTATION

(30) Priorität: 07.05.1999 DE 19921279
(43) Veröffentlichungstag der Anmeldung: 18.07.2001
(73) Patentinhaber: Aesculap AG & Co. KG, 78532 Tuttlingen (DE)
(72) Erfinder: KLEFFNER, Bernhard V., IBMT Fraunhofer-Institut, 66386 St. Ingbert (DE); MAYER, H. Michael, Orthopädische Klinik München, Harlachinger Str. 51, 81547 München (DE); WING, Charles, D-78573 Wurmlingen (DE)
(74) Vertreter: Boehme, Ulrich
(86) Internationale Anmeldenummer: PCT/EP2000/002253
(87) Internationale Veröffentlichungsnummer: WO 2000/067645

(56) Entgegenhaltungen:
- WO-A-96/11638
- DE-U- 29 908 259
- PATENT ABSTRACTS OF JAPAN vol. 95, no. 5, 30. Juni 1995 (1995-06-30) & JP 07 051277 A (HITACHI MEDICAL), 28. Februar 1995 (1995-02-28)
- "ORTHOPAEDIC SURGERY (Hall MICRO E system)" 1992 , ZIMMER HALL SURGICAL , CARPINTERIA, CALIFORNIA USA XP002141166 Prospekt, insbesondere "Surgitome E Drill" und "Fixation Drill"

## Beschreibung

Die Erfindung betrifft ein rotierendes chirurgisches Werkzeug zur Erzeugung einer Vertiefung in Knochenmaterial.

Zur Herstellung von Vertiefungen in Knochenmaterial sind die unterschiedlichsten rotierenden Werkzeuge bekannt, beispielsweise Bohrer, Fräser, Reibahlen, etc. All diesen Werkzeugen ist gemeinsam, daß mit ihnen Vertiefungen in verschiedenster Form in das Knochenmaterial eingebracht werden, beispielsweise Aufnahmebohrungen für Knochenschrauben. Es ist dabei von größter Wichtigkeit, daß diese Bohrungen und Vertiefungen in der gewünschten Weise im Knochen plaziert werden, da in vielen Fällen nur wenig Knochenmaterial zur Fixierung von Knochenschrauben und anderen Implantatteilen zur Verfügung steht. Beispielsweise ist es äußerst schwierig, im Wirbelsäulenbereich Pedikelschrauben so im Wirbelkörper zu plazieren, daß sie einerseits in der gewünschten Weise im Wirbelkörper festgelegt werden und andererseits keine Verletzungen hervorrufen.

Bisher war dies im wesentlichen unter Sicht des Operateurs durchzuführen oder allenfalls unter Röntgenkontrolle, und dies war unter Umständen mit einer erhöhten Strahlenbelastung für Patient und Operateur verbunden.

Aus der Veröffentlichung *Patent Abstracts of Japan* Vol. 95, Nr. 5, 30. Juni 1995 & JP-A-07 051277 ist es bekannt, ein rotierendes chirurgisches Werkzeug zur Erzeugung einer Vertiefung in Knochenmaterial mit einem separaten Ultraschallwandler zu überwachen, der die Schichtdicke des Knochens ermittelt.

Es ist Aufgabe der Erfindung, ein gattungsgemäßes Werkzeug so auszubilden, daß mit ihm Vertiefungen in Knochenmaterial kontrolliert in der gewünschten Position und Richtung erzeugt werden können.

Diese Aufgabe wird bei einem rotierenden chirurgischen Werkzeug der eingangs beschriebenen Art erfindungsgemäß dadurch gelöst, daß in ihm ein Ultraschallwandler angeordnet ist, der Ultraschallwellen aussenden und empfangen kann, und daß der Ultraschallwandler mit einem Ultraschallgenerator und einer Empfangseinrichtung verbindbar ist, die abhängig von der Stärke der vom Ultraschallwandler empfangenen Ultraschallstrahlung und der Zeitdauer zwischen Aussendung der Ultraschallstrahlung und dem Empfang reflektierter Ultraschallstrahlung Signale erzeugt, die ein Maß für die Beschaffenheit des Knochenmaterials in Ausstrahlungsrichtung sind.

Die vom Wandler im rotierenden Werkzeug ausgesandte Ultraschallstrahlung dringt in die umgebenden Knochenstrukturen ein und wird dort reflektiert, insbesondere an den Oberflächen des Knochenmaterials und an Inhomogenitäten im Knochenmaterial, beispielsweise an Flächen, an denen sich die Struktur des Knochenmaterials verändert. Die reflektierte Ultraschallstrahlung wird vom Wandler aufgefangen, und aus der Stärke des aufgefangenen Signals und der Zeit, die seit der Aussendung der Ultraschallstrahlung vergangen ist, lassen sich Angaben über die Beschaffenheit des an das Werkzeug angrenzenden Knochenmaterials machen, insbesondere über die Schichtdicke des Knochenmaterials und gegebenenfalls auch über Strukturänderungen des Knochenmaterials. Diese Information kann der Operateur nutzen, um die Position des Werkzeugs im Knochen zu überprüfen und damit die Lage der vom Werkzeug erzeugten Vertiefung.

Dabei können getrennte Wandler für das Aussenden und für das Empfangen der Ultraschallstrahlung vorgesehen sein, es ist aber auch möglich, die Ultraschallstrahlung mit demselben Wandler auszusenden und die reflektierte Strahlung anschließend wieder aufzunehmen. Dies läßt sich beispielsweise mit einem sogenannten Impuls-Echo-Verfahren durchführen.

Günstig ist es, wenn der Ultraschallwandler im Bereich des distalen Endes des Werkzeuges angeordnet ist, so daß von der vom Werkzeug ausgesandten Ultraschallstrahlung der Bereich vor dem distalen Ende des Werkzeugs "gesehen" wird. Dem Operateur wird somit eine Information darüber geliefert, wie das Knochenmaterial in Bearbeitungsrichtung beschaffen ist, und es wird ihm daher die Möglichkeit gegeben, die Vorschubrichtung des Werkzeugs entsprechend zu steuern.

Eine besonders günstige Ausgestaltung ergibt sich, wenn der Ultraschallwandler im Werkzeug derart angeordnet ist, daß seine Aussende- und Empfangsrichtung für die Ultraschallwellen gegenüber der Drehachse geneigt ist, beispielsweise mit einem Neigungswinkel zwischen 30 und 60°, insbesondere in der Größenordnung von etwa 45°. Die Ultraschallstrahlung wird bei einer solchen Ausgestaltung auf einem Kegelmantel ausgesandt, der sich in distaler Richtung öffnet, und auf diese Weise erhält der Operateur Informationen nicht nur genau in Vorschubrichtung des rotierenden Werkzeugs, sondern über den gesamten Drehwinkel des Werkzeugs in einem Bereich, der in distaler Richtung vor dem Werkzeug liegt. Dadurch ergibt sich eine optimale Orientierung über die noch zu bearbeitenden Knochenbereiche.

Bei einer bevorzugten Ausführungsform der Erfindung ist vorgesehen, daß das Werkzeug einen inneren Aufnahmeraum für den Ultraschallwandler aufweist, der mit einem im Werkzeug bis zu dessen proximalem Ende verlaufenden Kanal in Verbindung steht. Durch diesen Kanal können Anschlußleitungen für den Ultraschallwandler verlaufen.

Bei einer bevorzugten Ausführungsform ist das Werkzeug ein Bohrwerkzeug mit einer kegeligen Schneidfläche und der Ultraschallwandler ist im Bereich der kegeligen Schneidfläche angeordnet. Mit einem solchen Bohrer kann der Operateur während des Bohrvorgangs genau sehen, ob er beim weiteren Vortritt des Bohrers von allen Begrenzungen des Knochens einen ausreichenden Abstand hat, so daß die Bohrung mit Sicherheit im Knochenmaterial verläuft und nicht unerwünscht in Hohlräume oder anderes Gewebe eindringt.

Besonders günstig ist es, wenn das Werkzeug einen Sensor für seine Winkelstellung umfaßt und wenn der Sensor ein der Winkelstellung entsprechendes Signal der Empfangseinrichtung zuführt, die damit die Signale für die Knochenbeschaffenheit in Abhängigkeit von der Winkelstellung des Werkzeugs erzeugt. Damit erhält der Operateur ein umfassendes Bild der Beschaffenheit des Knochenmaterials vor dem Werkzeug, und zwar in allen Winkelrichtungen. Der Ultraschallwandler bildet gleichsam eine auf einem drehbaren Träger angeordnete Kamera, die den gesamten vor dem Werkzeug liegenden Bereich in allen Richtungen überstreicht.

Weiterhin kann vorgesehen sein, daß die Empfangseinrichtung ein optisches Anzeigegerät zugeordnet ist, welches die von der Empfangseinrichtung erzeugten Signale für die Knochenbeschaffenheit anzeigt. Auf einem solchen Anzeigegerät ist also direkt ablesbar, wie der Knochen in Ausstrahlungsrichtung der Ultraschallstrahlung beschaffen ist, welche Knochenwandstärke hier zur Verfügung steht und welche Strukturänderungen gegebenenfalls auftreten.

Besonders vorteilhaft ist es dabei, wenn auf dem optischen Anzeigegerät Querschnitte durch das Werkzeug und das angrenzende Knochenmaterial darstellbar sind, wobei die Beschaffenheit des Knochenmaterials durch die von der Empfangseinrichtung erzeugten Signale bestimmt wird. Diese Querschnitte zeigen gleichzeitig die von der Empfangseinrichtung erzeugten Signale an, die sich bei unterschiedlicher Winkelstellung des Werkzeugs ergeben, so daß man über den gesamten Winkelbereich gleichzeitig eine Information über die Knochenbeschaffenheit erhält.

Insbesondere kann dabei vorgesehen sein, daß die dargestellte Querschnittsfläche eine Kegelfläche ist, deren Achse mit der Drehachse des Werkzeugs zusammenfällt und die sich in distaler Richtung öffnet. Damit wird ein Anzeigebereich dargestellt, der den Bereich vor dem Werkzeug abtastet und somit die Knochenbeschaffenheit in dem Bereich anzeigt, in dem das Werkzeug erst in Zukunft eindringen wird.

Auf dem optischen Anzeigegerät können auch Querschnitte von Implantaten eingeblendet werden, die zeigen, in welcher Weise bestimmte Implantate nach der Implantation im Knochen angeordnet werden sollen. Der Operateur hat damit die Möglichkeit, bei Kenntnis der Implantatform und der gewünschten Lage des Implantats Vertiefungen, beispielsweise Bohrlöcher, so zu setzen, daß deren Lage mit der Form und der Position des Implantats korrespondiert. Die Abbildung des Implantats kann dabei aus einem Datenspeicher erzeugt werden, in denen die Darstellungsdaten für dieses Implantat gespeichert sind. Aus diesen Daten kann die Abbildung der Abbildung überlagert werden, die sich aus den Ultraschallsignalen ergibt.

Der Empfangseinrichtung kann auch eine beispielsweise optisch oder akustisch arbeitende Warneinrichtung zugeordnet sein, die den Operateur davor warnt, daß die Wandstärke des Knochenmaterials in dem zu bearbeitenden Bereich einen bestimmten Wert unterschreitet, daß also die Gefahr eines Durchbruchs besteht.

Die nachfolgende Beschreibung einer bevorzugten Ausführungsform der Erfindung dient im Zusammenhang mit der Zeichnung der näheren Erläuterung. Es zeigen:

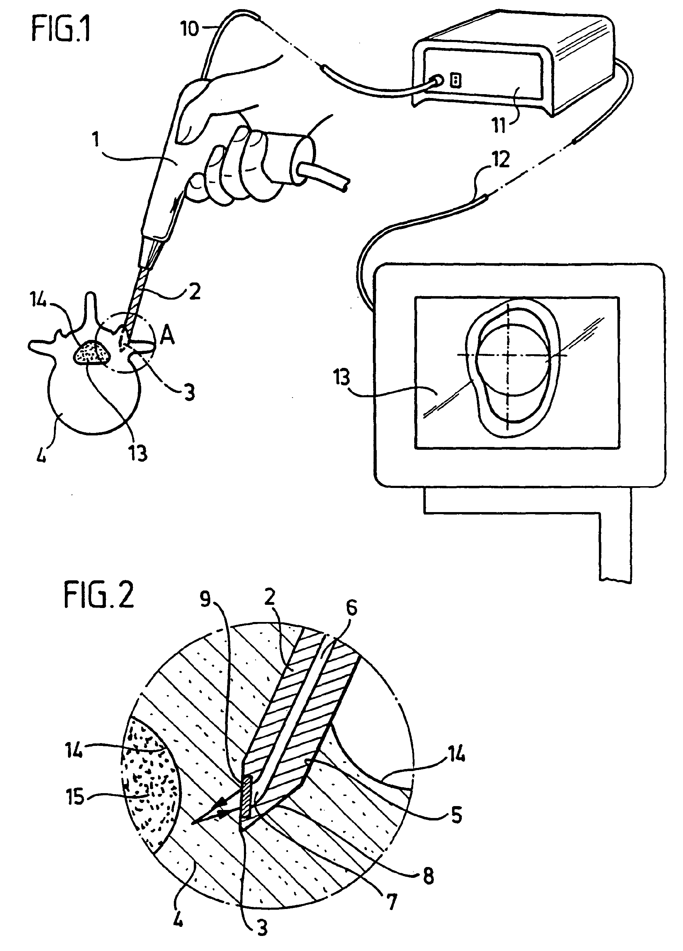

- Figur 1:: eine schematische Ansicht eines an einem Wirbelkörper angesetzten Bohrwerkzeugs mit einer Ultraschallüberwachungseinrichtung mit optischer Anzeige und
- Figur 2:: eine vergrößerte Schnittansicht im Bereich A in Figur 1 mit einem mit einem Ultraschallwandler ausgerüsteten Bohrwerkzeug.

Die Erfindung wird nachstehend am Beispiel eines Bohrwerkzeugs erörtert, grundsätzlich kann die Erfindung aber auch bei anderen drehenden Bearbeitungswerkzeugen verwendet werden, beispielsweise bei Fräsern, Reibahlen, Trephinen, etc., mit deren Hilfe durch Drehbewegung eine spanabhebende Bearbeitung des Knochenmaterials erfolgt, insbesondere zur Herstellung von Bohrungen und anderen Vertiefungen.

Eine chirurgische Handbohrmaschine 1 ist mit einem Spiralbohrer 2 bestückt, der durch einen Antrieb im Inneren der Handbohrmaschine 1 um seine Längsachse verdreht wird und mit seiner Spitze 3 in einem Knochen 4, im dargestellten Ausführungsbeispiel in einem Wirbelknochen im Pedikelbereich, eine Bohrung 5 erzeugt.

Im Gegensatz zu einem herkömmlichen Spiralbohrer ist dieser Spiralbohrer 2 mit einem vom proximalen Ende bis in den distalen Endbereich durchgehenden Innenkanal 6 versehen, der in einem Aufnahmeraum 7 endet, der sich seinerseits unmittelbar hinter der kegeligen Schneidfläche 8 des Spiralbohrers 2 befindet. In diesem Aufnahmeraum 7 ist ein Ultraschallwandler 9 angeordnet, der im wesentlichen senkrecht zur Schneidfläche 8 Ultraschallstrahlung abstrahlen und aus dieser Richtung auf ihn auftreffende Ultraschallstrahlung aufnehmen kann. Es kann sich dabei um einen einzigen Ultraschallwandler 9 handeln oder zwei getrennte Ultraschallwandler, die jeweils für Abstrahlung oder Aufnahme von Ultraschallstrahlung ausgebildet sind.

Der Ultraschallwandler ist über eine in der Zeichnung nicht näher dargestellte Leitung, die durch den Innenkanal 6 führt mit einer Leitung 10 verbunden, die von der Handbohrmaschine 1 ausgehend an einer Sende- und Empfangseinrichtung 11 endet, die ihrerseits über eine Leitung 12 mit einer Anzeigeeinrichtung 13 verbunden ist. Die Sende- und Empfangseinrichtung 11 kann beispielsweise als sogenannte Impuls-Echo-Anlage arbeiten und Ultraschallstrahlung mit Frequenzen in der Größenordnung von 15 MHz erzeugen und aufnehmen. Diese Ultraschallstrahlung wird in Form eines Impulses durch den Ultraschallwandler 9 in den umgebenden Knochen 4 ausgesandt und dort an Inhomogenitäten und an Grenzflächen reflektiert, beispielsweise an der Grenzfläche 14 zum Markraum 15 des Wirbelknochens.

Die reflektierte Strahlung trifft anschließend wieder auf den Ultraschallwandler 9 auf und wird dort in ein elektrisches Signal umgewandelt, das der Sende- und Empfangseinrichtung 11 zugeführt wird. Die Größe des der reflektierten Strahlung entsprechenden Signals hängt ab von der Stärke der Reflexion an die Inhomogenität, die Zeitdauer zwischen Aussendung des Impulses und Aufnahme der reflektierten Strahlung hängt ab von der Dicke des Knochenmaterials bis zu einer Grenzfläche und der Beschaffenheit des Knochenmaterials, da diese Beschaffenheit die Ausbreitungsgeschwindigkeit der Ultraschallstrahlung beeinflußt.

Auf diese Weise ist es möglich, bei Kenntnis des Aufbaus des Knochens festzustellen, in welchem Abstand Grenzflächen und Inhomogenitäten des Knochens von dem Ultraschallwandler angeordnet sind und zwar jeweils in Richtung der Ausstrahlung und des Empfangs des Ultraschallwandlers. Dieser Ultraschallwandler dreht sich zusammen mit dem Spiralbohrer 2 und kann daher bei der Aussendung und dem Empfang der Ultraschallstrahlung einen Kegelmantel überstreichen, der durch einen koaxial zu der Drehachse des Spiralbohrers 2 angeordneter und sich in distaler Richtung öffnenden Kegel gebildet wird. Die der Sende- und Empfangseinrichtung 11 zugeführten Reflexionssignale sind somit ein Maß für die Knochenbeschaffenheit und die Dicke des Knochenmaterials auf diesem Kegelmantel, also in einem Bereich, der dem Spiralbohrer 2 distal vorgelagert ist und in den der Spiralbohrer 2 bei weiterer Bearbeitung eintreten wird.

Die von der Sende- und Empfangseinrichtung 11 aufgenommenen Signale können auf der Anzeigeeinrichtung 13 so dargestellt werden, daß ausgehend von der Lage des Spiralbohrers 2 das von der Ultraschallstrahlung überstrichene Gebiet des Knochen um den Spiralbohrer 2 herum dargestellt wird, so daß der Operateur eine optische Anzeige dafür bekommt, wie das Knochenmaterial um den Spiralbohrer 2 herum in einem distal vor dem Spiralbohrer liegenden Bereich beschaffen ist, beispielsweise wie groß der Abstand ist zum nächsten Hohlraum oder zu einer Grenzfläche des Knochens ist. Dies ermöglicht dem Operateur den Spiralbohrer 2 so zu richten, daß er beim weiteren Eindringen in den Knochen 4 immer möglichst genau im Knochenmaterial zentriert wird und einen genügenden Abstand von den Grenzflächen des Knochens einhält. Außerdem ist es möglich, auf diese Weise den Spiralbohrer in Bereiche von besonders festem Knochenmaterial zu richten, da die reflektierten Ultraschallsignale auch Auskunft über die Beschaffenheit des Knochenmaterials geben, beispielsweise ändert sich die Fortpflanzungsgeschwindigkeit der Ultraschallwellen im Knochengewebe bei unterschiedlich strukturiertem Knochengewebe.

In der Spitze des Spiralbohrers 2 ist somit eine Ultraschallkamera mit Empfangsgerät eingebaut, die den Bereich beobachtet, der vor der Spitze des Spiralbohrers 2 angeordnet ist und die damit dem Operateur Informationen darüber bietet, in welche Knochenbereiche der Spiralbohrer 2 beim weiteren Eindringen gelangen wird.

## Patentansprüche

1. Rotierendes chirurgisches Werkzeug (2) zur Erzeugung einer Vertiefung in Knochenmaterial, **dadurch gekennzeichnet, daß** in ihm ein Ultraschallwandler (9) angeordnet ist, der Ultraschallwellen aussenden und empfangen kann, und daß der Ultraschallwandler (9) mit einem Ultraschallgenerator und einer Empfangseinrichtung (11) verbindbar ist, die abhängig von der Stärke der vom Ultraschallwandler (9) empfangenen Ultraschallstrahlung und der Zeitdauer von der Aussendung der Ultraschallstrahlung und dem Empfang reflektierter Ultraschallstrahlung Signale erzeugt, die ein Maß für die Beschaffenheit des Knochenmaterials (4) in Ausstrahlungsrichtung sind.

2. Werkzeug nach Anspruch 1, **dadurch gekennzeichnet, daß** der Ultraschallwandler (9) im Bereich des distalen Endes (3) des Werkzeugs (2) angeordnet ist.

3. Werkzeug nach einem der Ansprüche 1 oder 2, **dadurch gekennzeichnet, daß** der Ultraschallwandler (9) im Werkzeug (2) derart angeordnet ist, daß seine Aussende- und Empfangsrichtung für die Ultraschallwellen gegenüber der Drehachse geneigt ist.

4. Werkzeug nach Anspruch 3, **dadurch gekennzeichnet, daß** der Neigungswinkel zwischen 30° und 60° liegt.

5. Werkzeug nach einem der voranstehenden Ansprüche, **dadurch gekennzeichnet, daß** es einen inneren Aufnahmeraum (7) für den Ultraschallwandler (9) aufweist, der mit einem im Werkzeug (2) bis zu dessen proximalem Ende verlaufenden Kanal (6) in Verbindung steht.

6. Werkzeug nach einem der voranstehenden Ansprüche, **dadurch gekennzeichnet, daß** es ein Bohrwerkzeug (2) mit einer kegeligen Schneidfläche (8) ist und daß der Ultraschallwandler (9) im Bereich der kegeligen Schneidfläche (8) angeordnet ist.

7. Werkzeug nach einem der voranstehenden Ansprüche, **dadurch gekennzeichnet, daß** es einen Sensor für seine Winkelstellung umfaßt, und daß der Sensor ein der Winkelstellung entsprechendes Signal der Empfangseinrichtung (11) zuführt, die damit die Signale für die Knochenbeschaffenheit in Abhängigkeit von der Winkelstellung des Werkzeugs (2) erzeugt.

8. Werkzeug nach einem der voranstehenden Ansprüche, **dadurch gekennzeichnet, daß** die Empfangseinrichtung (11) mit einem optischen Anzeigegerät (13) verbindbar ist, welches die von der Empfangseinrichtung erzeugten Signale für die Knochenbeschaffenheit anzeigt.

9. Werkzeug nach Anspruch 8, **dadurch gekennzeichnet, daß** auf dem optischen Anzeigegerät (13) Querschnitte durch das Werkzeug (2) und das angrenzende Knochenmaterial (4) darstellbar sind, wobei die Beschaffenheit des Knochenmaterials (4) durch die von der Empfangseinrichtung (11) erzeugten Signale bestimmt wird.

10. Werkzeug nach Anspruch 9, **dadurch gekennzeichnet, daß** die dargestellte Querschnittsfläche eine Kegelfläche ist, deren Achse mit der Drehachse des Werkzeugs (2) zusammenfällt und die sich in distaler Richtung öffnet.

11. Werkzeug nach einem der Ansprüche 8 bis 10, **dadurch gekennzeichnet, daß** auf dem optischen Anzeigegerät zusätzlich Darstellungen von Implantaten sichtbar gemacht werden können.

12. Werkzeug nach einem der voranstehenden Ansprüche, **dadurch gekennzeichnet, daß** der Empfangseinrichtung (11) eine Warneinrichtung zugeordnet ist, die in Abhängigkeit von der Knochenstärke ein Warnsignal erzeugt.

## Claims

1. Rotating surgical tool (2) for producing a depression in bone material, **characterized in that** an ultrasonic transducer (9) is arranged in the surgical tool, the ultrasonic transducer being capable of emitting and receiving ultrasonic waves, and **in that** the ultrasonic transducer (9) is connectable to an ultrasonic generator and a receiver (11), the receiver generating signals in dependence upon the strength of the ultrasonic radiation received by the ultrasonic transducer (9) and upon the length of time between the emission of ultrasonic radiation and the reception of reflected ultrasonic radiation, the signals being a measure of the condition of the bone material (4) in the direction of emission.

2. Tool in accordance with claim 1, **characterized in that** the ultrasonic transducer (9) is arranged in the area of the distal end (3) of the tool. (2).

3. Tool in accordance with claim 1 or 2, **characterized in that** the ultrasonic transducer (9) is arranged in the tool (2) such that the direction in which it emits and receives the ultrasonic waves is at an incline to the axis of rotation.

4. Tool in accordance with claim 3, **characterized in that** the angle of inclination lies between 30° and 60°.

5. Tool in accordance with any one of the preceding claims, **characterized in that** it comprises an inside receiving space (7) for the ultrasonic transducer (9), which is in communication with a channel (6) extending in the tool (2) as far as the proximal end thereof.

6. Tool in accordance with any one of the preceding claims, **characterized in that** it is a drill (2) with a conical cutting surface (8), and **in that** the ultrasonic transducer (9) is arranged in the area of the conical cutting surface (8).

7. Tool in accordance with any one of the preceding claims, **characterized in that** it includes a sensor for its angular position, and **in that** the sensor feeds a signal corresponding to the angular position to the receiver (11) which thus generates the signals for the condition of the bone in dependence upon the angular position of the tool (2).

8. Tool in accordance with any one of the preceding claims, **characterized in that** the receiver (11) is connectable to an optical display device (13) which indicates the signals generated by the receiver for the condition of the bone.

9. Tool in accordance with claim 8, **characterized in that** cross sections through the tool (2) and the adjacent bone material (4) are able to be illustrated on the optical display device (13), and the condition of the bone material (4) is determined by the signals generated by the receiver (11).

10. Tool in accordance with claim 9, **characterized in that** the illustrated cross-sectional surface is a conical surface which opens in the distal direction, and the axis of which coincides with the axis of rotation of the tool (2).

11. Tool in accordance with any one of claims 8 to 10, **characterized in that**, in addition, illustrations of implants are able to be made visible on the optical display device.

12. Tool in accordance with any one of the preceding claims, **characterized in that** a warning device which generates a warning signal in dependence upon the strength of the bone is associated with the receiver (11).

## Revendications

1. Outil chirurgical rotatif (2) destiné à usiner un creux dans la matière osseuse, **caractérisé en ce que**, dans ledit outil, est agencé un transducteur d'ultrasons (9) qui peut émettre et recevoir des ondes ultrasonores et **en ce que** le transducteur d'ultrasons (9) peut être relié à un générateur d'ultrasons et à un dispositif de réception (11) qui, en fonction de l'intensité du rayonnement ultrasonore reçu par le transducteur d'ultrasons (9) et de l'intervalle de temps entre l'émission du rayonnement ultrasonore et la réception du rayonnement ultrasonore réfléchi, génère des signaux qui sont un critère de la constitution de la matière osseuse (4) dans la direction du rayonnement.

2. Outil selon la revendication 1, **caractérisé en ce que** le transducteur (9) est agencé dans la zone de l'extrémité distale (3) de l'outil (2).

3. Outil selon la revendication 1 ou 2, **caractérisé en ce que** le transducteur (9) est agencé dans l'outil (2) de telle sorte que son dispositif d'émission et de réception des ondes ultrasonores est incliné par rapport à l'axe de rotation.

4. Outil selon la revendication 3, **caractérisé en ce que** l'angle d'inclinaison se situe entre 30° et 60°.

5. Outil selon l'une quelconque des revendications précédentes, **caractérisé en ce qu'**il comporte un logement (7) intérieur pour le transducteur (9), lequel communique avec un conduit (6) qui s'étend dans l'outil (2) jusqu'à l'extrémité proximale de celui-ci.

6. Outil selon l'une quelconque des revendications précédentes, **caractérisé en ce qu'**il s'agit d'un perceuse (2) avec une surface de coupe (8) conique et **en ce que** le transducteur d'ultrasons (9) est agencé dans la zone de la surface de coupe (8) conique.

7. Outil selon l'une quelconque des revendications précédentes, **caractérisé en ce qu'**il comporte un capteur pour sa position angulaire et **en ce que** le capteur transfère un signal correspondant à la position angulaire vers le dispositif de réception (11), qui génère ainsi, en fonction de la position angulaire de l'outil (2), les signaux relatifs à la constitution de l'os.

8. Outil selon l'une quelconque des revendications précédentes, **caractérisé en ce que** le dispositif de réception (11) peut être relié à un écran optique (13), qui affiche les signaux relatifs à la constitution de l'os, générés par le dispositif de réception.

9. Outil selon la revendication 8, **caractérisé en ce que** des coupes transversales de l'outil (2) et de la matière osseuse (4) adjacente peuvent être représentées sur l'écran optique (13), la constitution de la matière osseuse (4) étant déterminée par les signaux générés par le dispositif de réception (11).

10. Outil selon la revendication 9, **caractérisé en ce que** la surface transversale représentée est une surface conique, dont l'axe coïncide avec l'axe de rotation de l'outil (2) et qui s'ouvre dans la direction distale.

11. Outil selon l'une quelconque des revendications 8 à 10, **caractérisé en ce que** des représentations d'implants peuvent également être reproduites sur l'écran optique.

12. Outil selon l'une quelconque des revendications précédentes, **caractérisé en ce qu'**un système d'alarme est associé au dispositif de réception (11), lequel génère un signal d'alarme en fonction de l'épaisseur de l'os.
